(19) Europäisches Patentamt / European Patent Office / Office européen des brevets

(11) **EP 3 677 243 A1**

(12) **EUROPEAN PATENT APPLICATION**
published in accordance with Art. 153(4) EPC

(43) Date of publication:
**08.07.2020 Bulletin 2020/28**

(51) Int Cl.:
*A61K 8/02* (2006.01)     *A61K 8/92* (2006.01)
*A61K 8/67* (2006.01)     *A61Q 19/08* (2006.01)

(21) Application number: **18851881.5**

(22) Date of filing: **28.08.2018**

(86) International application number:
**PCT/KR2018/009918**

(87) International publication number:
**WO 2019/045413 (07.03.2019 Gazette 2019/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.08.2017 KR 20170108700**

(71) Applicant: **Daeduck Lab Co., Ltd.**
**Yuseong-gu, Daejeon 34024 (KR)**

(72) Inventor: **JHUN, Hyun Pyo**
**Daejeon 34200 (KR)**

(74) Representative: **Adam, Holger**
**Kraus & Weisert**
**Patentanwälte PartGmbB**
**Thomas-Wimmer-Ring 15**
**80539 München (DE)**

(54) **COSMETIC COMPOSITION INCLUDING PHYSIOLOGICALLY ACTIVE ANTIOXIDANT, AND DOUBLE-LAYER COSMETIC INCLUDING SAME**

(57) The present invention relates to: a cosmetic composition including a physiologically active antioxidant such as vitamin C or the like; a double-layer cosmetic including same; and a method for inhibiting oxidation of the antioxidant, wherein oxidation of the physiologically active antioxidant can be inhibited by covering a surface layer of an aqueous phase containing the physiologically active antioxidant with an oil phase to prevent exposure to oxygen in the atmosphere, and the process thereof is not complicated and thus has the effect of being efficient. In addition, the method for inhibiting oxidation of a cosmetic composition including a physiologically active antioxidant according to the present invention has an advantage in that the method can be used together with existing techniques, such as a technique for chemically stabilizing the physiologically active antioxidant, and a encapsulation technique by which the physiologically active antioxidant is stabilized by being trapped inside a capsule.

【FIG. 1】

## Description

[Technical Field]

The present invention relates to a cosmetic composition including a physiologically active antioxidant and double-layer cosmetics including the same.

[Background Art]

An antioxidant generally used in cosmetics refers to a compound that has various antioxidant characteristics, for example, of reducing or preventing a phenomenon, in which the skin function and structure damaged by an action of oxidation is destroyed and exhausted, to considerable extents, supporting the physiological activity in the human body, preventing aging of the human body, and the like. Types of physiologically active antioxidants include various compounds such as vitamins, polyphenols, and the like. Among vitamins, vitamin C is particularly known as a superior antioxidant.

Specifically, vitamin C is a lactone having 6 carbon atoms and is soluble in water. It is known that plants and most animals can synthesize vitamin C *in vivo*, but humans, primates, guinea pigs, fruit bats, and some species of birds and fishes cannot synthesize vitamin C. Vitamin C includes ascorbic acid in a reduced form and dehydroascorbic acid in an oxidized form, wherein the two substances may be converted to each other.

Vitamin C has been used for cosmetics for a long time, and is one of the raw materials notified as the functional material for whitening by the Ministry of Food and Drug Safety because it serves to remove active oxygen detrimental to the skin due to an antioxidant action and is excellent in inhibiting production of melanin pigments. Also, vitamin C is used as an anti-wrinkle material because it aids in the biosynthesis of collagen.

However, because vitamin C is chemically unstable, it is easily degraded by sunlight, heat, and surrounding pH. In particular, because vitamin C is oxidized by reaction with oxygen in the air, the vitamin C has actual limitations in showing normal efficacy when the vitamin C is used for cosmetics. In recent years, research on relatively stable chemical derivatives of vitamin C, research on types of capsules coated with particles including vitamin C or internally charged with vitamin C, and the like have been conducted in order to inhibit the oxidation of vitamin C.

Many types of the vitamin C derivatives including sodium ascorbyl phosphate, ascorbyl palmitate, retinyl ascorbate, and the like have been developed and used to overcome the lack of stability of vitamin C. However, it was reported that the synthetic derivatives such as representative vitamin C derivatives (such as ascorbyl phosphate), fat-soluble derivatives (such as ascorbyl palmitate), and the like have poor permeability to the skin. That is, it can be seen that using vitamin C having a natural structure to prepare vitamin C-containing cosmetics is more effective for the skin, compared to using the synthetic derivatives. However, in the prior art, when the vitamin C itself is used for the vitamin C-containing cosmetics, the vitamin C has many limitations due to its low oxidative stability, as described above. Therefore, the use of the synthetic derivatives of vitamin C was an inevitable option. When vitamin C itself is used, for example, methods of storing vitamin C as a main ingredient in a special ampule, and the like have been used to prevent oxidation of vitamin C during storage. However, such a method has drawbacks in that an increase in unit cost of products may be caused, and the products are obliged to be used for disposable purposes only.

In particular, because oxygen in the atmosphere enters an ampule or a cosmetic container including the cosmetic composition containing vitamin C immediately after the ampule or the cosmetic container is opened, vitamin C is oxidized by frequent exposure to oxygen due to the characteristics of the cosmetic packaging that is designed to frequently open and close a lid of the container. Eventually, it is difficult to avoid accelerating the oxidation of the vitamin C derivatives in use.

[Disclosure]

[Technical Problem]

An object of the present invention is to provide a cosmetic composition including a physiologically active antioxidant capable of effectively inhibiting denaturation of the physiologically active antioxidant by preventing exposure to oxygen in the atmosphere to inhibit oxidation of the physiologically active antioxidant; and double-layer cosmetics including the same.

Another object of the present invention is to provide a method for inhibiting oxidation of a physiologically active antioxidant capable of effectively inhibiting denaturation of the physiologically active antioxidant even when a process thereof is not complicated but is easy compared to the conventional techniques for stabilizing the physiologically active antioxidant.

Still another object of the present invention is to provide a technique for inhibiting oxidation of the physiologically active antioxidant capable of being applied together with the prior-art techniques such as a technique for chemically stabilizing the physiologically active antioxidant, an encapsulation technique for encapsulating the physiologically active antioxidant to stabilize the physiologically active antioxidant, and the like; a cosmetic composition to which the technique is applied; and double-layer cosmetics including the same.

[Technical Solution]

In one general aspect, a cosmetic composition including a physiologically active antioxidant, double-layer cosmetics including the same, and a method for inhibiting oxidation of the antioxidant are provided.

The cosmetic composition including the physiologically active antioxidant according to the present invention includes an aqueous phase including the physiologically active antioxidant; and an oil phase coming into contact with the entire upward surface layer of the aqueous phase, wherein the surface layer of the aqueous phase and a lower layer of the oil phase come into contact with each other to form an interface therebetween. That is, the surface layer of the aqueous phase may be covered with the lower layer of the oil phase to inhibit oxidation of the physiologically active antioxidant in the aqueous phase.

In the present invention, the cosmetics having the cosmetic composition accommodated in a case may be provided. For the cosmetics, the cosmetic composition may be accommodated in the case, the aqueous phase may come into contact with an inner bottom of the case, and one side of the aqueous phase may come into contact with an inner side of the case. Therefore, the entire upward surface layer of the aqueous phase may be covered with the oil phase to inhibit oxidation of the physiologically active antioxidant in the aqueous phase.

In addition, the method for inhibiting oxidation of the physiologically active antioxidant according to the present invention may include: disposing an oil phase on an aqueous phase including the physiologically active antioxidant. In this case, the oxidation of the physiologically active antioxidant in the aqueous phase may be inhibited by blocking the physiologically active antioxidant from coming into contact with oxygen in the atmosphere since a surface layer of the aqueous phase and a lower layer of the oil phase come into contact with each other to form an interface therebetween.

According to an exemplary embodiment of the present invention, preferably, the aqueous phase and the oil phase may not each independently substantially include a surfactant. As one non-limiting example, the aqueous phase and the oil phase may each independently further include the surfactant. When the aqueous phase and the oil phase each independently further include the surfactant, the surfactant may be included at 10% by weight or less, based on the total weight of the aqueous phase or the oil phase.

According to an exemplary embodiment of the present invention, the oil phase may have an oxygen solubility of 4.5 $\mu$mol/g or less at 1 atmospheric pressure (atm) and 25°C.

According to an exemplary embodiment of the present invention, an oil of the oil phase may satisfy the following Expression 1. In the following Expression 1, $N_O$ represents the number of oxygen atoms in the molecule, and $N_C$ represents the number of carbon atoms in the molecule.

[Expression 1]

$$N_O / N_C \leq 0.1$$

According to an exemplary embodiment of the present invention, the oil phase may further include a fat-soluble derivative of vitamin C.

According to an exemplary embodiment of the present invention, the physiologically active antioxidant may include vitamin C or a derivative thereof.

According to an exemplary embodiment of the present invention, the vitamin C or the derivative thereof may include any one or two more selected from ascorbic acid; a water-soluble derivative of ascorbic acid; a capsule having ascorbic acid collected therein; a capsule having a water-soluble derivative of ascorbic acid collected therein; and the like.

According to an exemplary embodiment of the present invention, the physiologically active antioxidant may include any one or two more selected from a carotenoid-based compound, a flavonoid-based compound, an isoflavone-based compound, a compound based on vitamins other than vitamin C, and the like.

[Advantageous Effects]

A cosmetic composition including a physiologically active antioxidant according to the present invention, and double-layer cosmetics including the same have an advantage in that a surface layer of an aqueous phase containing the physiologically active antioxidant can be covered with an oil phase to prevent exposure to oxigene in the atmosphere, thereby inhibiting oxidation of the physiologically active antioxidant to effectively inhibit denaturation of the physiologically active antioxidant.

A method for inhibiting oxidation of the physiologically active antioxidant according to the present invention can be effective in effectively inhibiting denaturation of the physiologically active antioxidant even when a process thereof is not complicated but is easy compared to the conventional techniques for stabilizing the physiologically active antioxidant.

The method for inhibiting oxidation of the physiologically active antioxidant according to the present invention has an advantage in that the method can be applied together with the prior-art techniques such as a technique for chemically stabilizing the physiologically active antioxidant, an encapsulation technique for encapsulating the physiologically active antioxidant to stabilize the physiologically active antioxidant, and the like.

Although the effects are not explicitly mentioned in the present invention, the effects described in this specification, which are expected by the technical features of the present invention, and provisional effects thereof are handled as described above in the specification of the present invention.

[Description of Drawings]

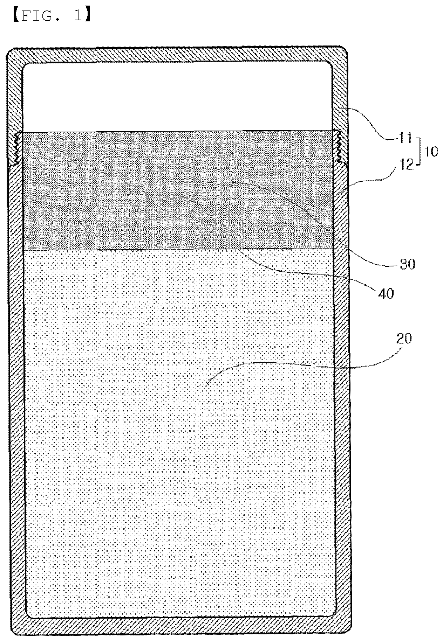

FIG. 1 is a schematic diagram showing a cosmetic case accommodating a cosmetic composition including vitamin C or a derivative thereof according to an exemplary embodiment of the present invention.

[Best Mode]

Hereinafter, a cosmetic composition including a physiologically active antioxidant according to the present invention, double-layer cosmetics including the same, and a method for inhibiting oxidation of the antioxidant will be described in detail with reference to the accompanying drawings.

The drawings presented in the present invention are shown as one example to sufficiently provide the scope of the present invention to those skilled in the art. Therefore, it should be understood that the present invention may be embodied in various forms, and is not intended to be limiting in the drawings presented hereinbelow. In this case, the drawings may be shown in an exaggerated manner to make the scope of the present invention more clearly apparent.

Also, unless otherwise defined, the technical and scientific terms used herein have the same meaning as commonly understood by one of ordinary skill in the art to which the present invention belongs. In the following description and the accompanying drawings, a description of known functions and configurations, which may unnecessarily obscure the subject matter of the present invention, will be omitted.

In addition, the singular forms "a," "an" and "the" unclearly used herein are intended to include the plural forms as well, unless the context clearly indicates otherwise.

Further, the units of "%" or "percentage" used without any particular comments in the present invention refer to a percent (%) by weight or weight percentage.

According to the present invention, a cosmetic composition including a physiologically active antioxidant, double-layer cosmetics including the same, and a method for inhibiting oxidation of the physiologically active antioxidant may be provided.

The cosmetic composition including a physiologically active antioxidant according to the present invention includes an aqueous phase including the physiologically active antioxidant; and an oil phase coming into contact with the entire upward surface layer of the aqueous phase, wherein a surface layer of the aqueous phase and a lower layer of the oil phase come into contact with each other to form an interface therebetween. In this case, the surface layer of the aqueous phase is covered with the lower layer of the oil phase to inhibit oxidation of the physiologically active antioxidant in the aqueous phase. Specifically, as shown as one example in FIG. 1, the oxidation of the physiologically active antioxidant in the aqueous phase is inhibited by blocking the physiologically active antioxidant from coming into contact with oxygen in the atmosphere since the surface layer of the aqueous phase and the lower layer of the oil phase come into contact with each other to form an interface therebetween.

That is, the cosmetic composition including the physiologically active antioxidant according to the present invention is characterized in that, because the cosmetic composition maintains a structure in which an interface layer is formed between the aqueous phase and the oil phase, the oil phase blocks or inhibits the physiologically active antioxidant disposed in the aqueous phase from coming into contact with oxygen in the atmosphere.

The physiologically active antioxidant is not limited as long as it is a compound used in the cosmetic composition, that is, a water-soluble compound that can alleviate or prevent oxidation of the skin. Specifically, the physiologically active antioxidant may include any one or two more selected from water-soluble vitamin-based compounds such as vitamin C, vitamins B (B1, B2, B6, B12, and the like), niacin, biotin, folic acid, pantothenic acid, vitamin G (glutathione), and the like; flavonoid-based compounds such as anthocyanin, catechin, resveratrol, proanthocyanidin, and the like; natural extracts; derivatives thereof; and the like. In addition, water-soluble substances having antioxidant characteristics may be used without being limited to the compound.

In the physiologically active antioxidant, when ascorbic acid (i.e., vitamin C) or a derivative thereof is applied to the present invention, the oxidation may be more effectively inhibited. Specifically, because vitamin C is chemically more unstable than other antioxidants, the vitamin C may be easily degraded by sunlight, heat, and surrounding pH. In particular, because vitamin C is easily oxidized by reaction with oxygen in the air, the vitamin C has actual limitations in normally showing the efficacy when the vitamin C is used for cosmetics. However, when the technique of the present invention is applied, oxidation of the vitamin C or the derivative thereof may be more effectively inhibited.

The derivative of vitamin C may refer to a compound synthesized for the purpose of improving characteristics (function, efficacy, and the like), stability, and the like of vitamin C, adding other characteristics, or the like; or a compound having a structure and characteristics similar to vitamin C. Examples of such a derivative of vitamin C may include ascorbyl phosphate, ascorbyl palmitate, retinyl ascorbate, and the like. In addition, various derivatives of vitamin C known in the art may also be used.

A weight ratio of the aqueous phase and the oil phase is desirable as long as the surface layer of the aqueous phase is covered with the oil phase so that the aqueous phase is not exposed to oxygen in the atmosphere. In this case, as the ratio of the oil phase increases, a content of the physiologically active antioxidant to be protected may be relatively reduced. When the ratio of the oil phase is too low, an effect of preventing the oxidation caused by the contact of the physiologically active antioxidant with oxygen in the air may be relatively lowered. As one specific example, the weight ratio or volume ratio of the aqueous phase and the oil phase may be in a range of 100:0.1 to 50, preferably in a range of 100:1 to 10. However, it should be understood that this is merely one preferred example, but is not intended to limit the present invention.

According to an exemplary embodiment of the present invention, the aqueous phase and the oil phase may not each independently substantially include a surfactant. As one non-limiting example, the aqueous phase and the oil phase may each independently further include the surfactant. In this case, the surfactant is desirable as long as it does not destroy a double layer of the aqueous phase and the oil phase. In this case, the surfactant may be included at a sufficient content so as not to destroy an interface layer between the aqueous phase and the oil phase and not to emulsify the interface layer as well. For example, the surfactant may be included at 10% by weight or less, preferably 2% by weight or less, and more preferably 0.001 to 1% by weight, based on the total weight of the aqueous phase or the oil phase.

The oil phase should have a sufficient hydrophilic lipophilic balance (HLB) value so as not to destroy a double layer of the aqueous phase and the oil phase. For example, the HLB value of the oil phase may be less than or equal to 3, preferably 2.

It is desirable that the oil phase has an oxygen solubility of 4.5 $\mu$mol/g or less, preferably 1.0 $\mu$mol/g or less, and more preferably 0.0001 to 0.5 $\mu$mol/g at 1 atm and 25°C. In this case, in the $\mu$mol/g, the unit of g represents a weight of the oil in the oil phase, and the unit of mol represents the number of moles of oxygen dissolved in the oil phase. When the oil phase satisfies this requirement, oxygen gas in the atmosphere may be effectively inhibited from being dissolved in the aqueous phase to minimize a problem in which the oxygen gas in the atmosphere flows through the oil phase to oxidize the physiologically active antioxidant included in the aqueous phase.

Also, when oils such as hydrocarbon; or a derivative thereof substituted with oxygen or nitrogen atom are used as the oil of the oil phase, it is desirable that the oil of the oil phase satisfies the following Expression 1, specifically satisfies the following Expression 2. In the following Expressions 1 and 2, $N_O$ represents the number of oxygen atoms in the molecule, and $N_C$ represents the number of carbon atoms in the molecule.

$$[\text{Expression 1}]$$

$$N_O/N_C \le 0.1$$

$$[\text{Expression 2}]$$

$$0 \le N_O/N_C \le 0.1$$

More preferably, it is more desirable that the oil satisfies the following Expression 3, and specifically satisfies the following Expression 4. In the following Expressions 3 and 4, $N_O$ represents the number of oxygen atoms in the molecule, and $N_C$ represents the number of carbon atoms in the molecule.

$$[\text{Expression 3}]$$

$$N_O/N_C \le 0.05$$

$$[\text{Expression 4}]$$

$$0 \le N_O/N_C \le 0.05$$

When the oil satisfies this requirement, the surfactant activity of the oil phase with respect to the aqueous phase may be lowered. Therefore, an interface may be formed between the oil phase and the aqueous phase to stably maintain layers separated from each other, thereby remarkably improving structure stability of the interface layer. Therefore, it is possible to minimize a problem in which the physiologically active antioxidant included in the aqueous phase is oxidized when the physiologically active antioxidant is exposed to oxygen gas in the atmosphere.

Oils that are present in a liquid phase at an ambient temperature commonly used for cosmetics may be used as the oil of the oil phase. In this case, the oils are desirable as long as they can form an interface with a surface layer of the aqueous phase without being mixed with the aqueous phase. As one preferred example, the oil of the oil phase may include any one or two more selected from hydrocarbon-based oils such as squalane, mineral oil, and the like; ester-based oils such as isopropyl palmitate, 2-octyldodecyl mirystate, isopropyl myristate, butyloctyl salicylate, cetyl octanoate, cetyloctyl hexanoate, coco-caprylate/caprate, decyl cocoate, isostearyl isostearate, pentaerythrityl tetraethylhexanoate, dicaprylyl carbonate, and the like; vegetable oils such as Caster oil, olive oil, Jojoba oil, avocado oil, macadamia oil, meadowfoam seed oil, Shea butter oil, mango butter oil, *Theobroma grandiflorum* seed butter oil, refined grapeseed oil, rosehip oil, safflower oil, peach kernel oil, sunflower seed oil, and the like; animal oils such as mink oil, horse oil, fish oil, and the like; silicone oils such as phenyl trimethicone, cyclomethicone, dimethicone, cyclopentasiloxane, decamethylcyclopentasiloxane, and the like; and fluorine-based oils such as perfluorinated polyether, polyoxyperfluoroalkane, and the like. Preferably, it is desirable to use an oil, which does not contain oxygen or contains a relatively small content of oxygen, such as squalane, mineral oil, and the like, in terms of further reducing a risk of bringing oxygen in the atmosphere into contact with the aqueous phase. However, this is merely one preferred example, and oils that may form a double layer without being substantially mixed with the aqueous phase are not limited. In this case, various types of oils may be used.

The oil phase may further include a fat-soluble derivative of vitamin C. Because the fat-soluble derivative of vitamin C has excellent oxidative stability upon exposure to oxygen in the atmosphere, compared to vitamin C, the fat-soluble derivative of vitamin C may be contained in the oil phase that may be directly exposed to the oxygen gas in the atmosphere. In particular, when the physiologically active antioxidant includes vitamin C or a derivative thereof, the fat-soluble derivative of vitamin C may be further included in the oil phase, thereby further enhancing an effect of vitamin C in the cosmetic composition including the aqueous phase and the oil phase.

The fat-soluble derivative of vitamin C may include various fat-soluble derivatives of vitamin C known in the art, for example, ascorbyl palmitate, and the like, but the present invention is not limited thereto. For example, various fat-soluble derivatives of vitamin C known in the art may be used. Also, as one non-limiting example, the oil phase may include a fat-soluble physiologically active antioxidant, for example, a carotenoid-based compound such as betacarotene, lycopene, lutein, and the like; an isoflavone-based compound such as genistein, daidzein, and the like; a derivative thereof; or the like.

The composition according to the present invention has an advantage in that the composition may be applied together with the prior-art stabilization techniques such as a technique for chemically stabilizing the physiologically active antioxidant, an encapsulation technique for encapsulating the physiologically active antioxidant to stabilize the physiologically active antioxidant, and the like. That is, a synergy effect of inhibiting oxidation of the physiologically active antioxidant caused by exposure of the physiologically active antioxidant to oxygen in the atmosphere is realized by applying a simple process of containing the physiologically active antioxidant, to which such conventional techniques for inhibiting the oxidation are applied, in the aqueous phase of the cosmetic composition, which has a structure in which an interface is formed between the aqueous phase and the oil phase according to the present invention, along with the method of the present invention.

Specifically, the physiologically active antioxidant may include any one or two more selected from a physiologically active antioxidant; a water-soluble derivative of the physiologically active antioxidant; a capsule having the physiologically active antioxidant collected therein; a capsule having the water-soluble derivative of the physiologically active antioxidant collected therein; and the like. In this case, the aforementioned various compounds may be used as the physiologically active antioxidant. More preferably, it is desirable to use vitamin C as the physiologically active antioxidant in terms of having a relatively superior effect of inhibiting the oxidation.

According to the present invention, the cosmetics including the cosmetic composition accommodated in a case may be provided, as described above. For the cosmetics according to an exemplary embodiment of the present invention, specifically, the cosmetic composition is accommodated in the case, the aqueous phase comes into contact with an inner bottom of the aqueous phase, and one side of the aqueous phase comes into contact with an inner side of the case, as shown as one example in FIG. 1. Therefore, the entire upward surface layer of the aqueous phase is covered with the oil phase to inhibit oxidation of the physiologically active antioxidant in the aqueous phase.

A material of the case is not limited, and may, for example, be selected from glass, a metal, plastics, ceramics, and the like. In addition, various substances may be used as long as they are materials that may accommodate a liquid-phase substance. As one non-limiting example, the material of the case may be a material having a low ultraviolet (UV) transmittance in terms of preventing oxidation of the physiologically active antioxidant caused by exposure to UV rays. However, it should be understood that this is substantially described as one preferred example, but is not intended to limit the present invention.

According to an exemplary embodiment of the present invention, when a double layer may be formed by forming an interface between the aqueous phase and the oil phase without mixing the aqueous phase and the oil phase with each other, known compounds used for various cosmetic compositions may be included in the aqueous phase or the oil phase.

For example, the compound may include any one or two more selected from a compound having a whitening effect, a compound having an anti-wrinkle effect, a compound having a moisturizing effect, and the like. When the cosmetic composition of the present invention forms a double layer by forming an interface between the aqueous phase and the oil phase without mixing the aqueous phase and the oil phase with each other, such compounds may be properly used because the compounds are widely known in the technical field of cosmetic compositions.

As one specific example, the compound having a whitening effect may include any one or two more selected from arbutin, ethyl ascorbyl ether, an oil soluble *Glycyrrhiza uralensis* extract, ascorbyl glucoside, magnesium ascorbyl phosphate, niacinamide, alpha-bisabolol, ascorbyl tetraisopalmitate, albumin, and the like. However, it should be understood that this is merely one specific example, but is not intended to limit the present invention.

As one specific example, the compound having an anti-wrinkle effect may include any one or two more selected from adenosine, retinol, collagen, retinyl palmitate, an epidermal growth factor, a fibroblast growth factor, and the like. However, it should be understood that this is merely one specific example, but is not intended to limit the present invention.

As one specific example, the compound having a moisturizing effect may include any one or two more selected from hyaluronic acid, glycerin, butylene glycol, dipropylene glycol, sorbitol, xylitol, sodium hyaluronate, a glyceryl acrylate/acrylic acid copolymer, an *Estern prickly pear* extract, and an aloe extract. However, it should be understood that this is merely one specific example, but is not intended to limit the present invention.

Hereinafter, the present invention will be described in detail with reference to exemplary embodiments thereof. However, it should be understood that the following examples are given only for the purpose of describing the present invention in further detail, and are not intended to limit the scope of the present invention.

[Example 1]

As shown in FIG. 1, a first mixed solution including 5% by weight of vitamin C (L-ascorbic acid, Junsei, Japan) and 95% by weight of deionized water as an aqueous phase was added into a cosmetic case at an ambient temperature and a normal pressure, and a second mixed solution including squalane ($N_O/N_C$ = 0) as an oil phase was added to an upper portion of the first mixed solution to prepare a cosmetic composition including vitamin C. In this case, a weight ratio of the first mixed solution and the second mixed solution was 8:2.

[Example 2]

This example was performed in the same manner as in Example 1, except that cetyl octanoate oil ($N_O/N_C$ =0.083) was used instead of the squalane as the oil phase of the second mixed solution used in Example 1.

[Example 3]

This example was performed in the same manner as in Example 1, except that jojoba oil ($N_O/N_C$ =0.048) was used instead of the squalane as the oil phase of the second mixed solution used in Example 1.

[Example 4]

This example was performed in the same manner as in Example 1, except that the second mixed solution used in Example 1 further included 5% by weight of ascorbyl palmitate, which was a fat-soluble derivative of vitamin C, based on the total weight of the second mixed solution.

[Example 5]

This example was performed in the same manner as in Example 1, except that capric/caprylic triglyceride ($N_O/N_C$ = 0.21) was used instead of the squalane as the oil phase of the second mixed solution used in Example 1.

[Comparative Example 1]

This example was performed in the same manner as in Example 1, except that the oil phase was excluded without using the second mixed solution used in Example 1.

[Experimental Example 1] Analysis of oxidation characteristics of vitamin C

To analyze oxidation characteristics of vitamin C in the aqueous phase of each of the cosmetic composition prepared in Examples 1 to 5 and Comparative Example 1, a change in content of vitamin C by exposure to oxygen in the atmosphere was measured for 3 hours using spectrometry.

Specifically, a lid (an upper case) of a cosmetic case accommodating the cosmetic composition was opened while being exposed to UV rays for oxidation conditions so that the cosmetic composition was exposed to oxygen in the atmosphere for 3 hours. After 3 hours, a content ratio of vitamin C after exposure to oxygen with respect to the total initial weight of vitamin C was measured using a BOD incubator (MIR-253, Sanyo, Japan) in a state in which the temperature was accurately maintained at 30°C. In this case, a content of vitamin C was analyzed by a method of plotting a calibration curve at the maximum absorption wavelength of 245 nm using a UV-visible spectrophotometer (UV-1700, Simadzu, Japan).

As a result, the content ratio of vitamin C after the exposure to oxygen with respect to the initial content of vitamin C was 99% in the case of Examples 1 to 4 and was 89% in the case of Example 5. On the contrary, it can be seen that the content ratio of vitamin C was 31% in the case of Comparative Example 1 having no oil layer, indicating that the content ratio of vitamin C after the exposure to oxygen with respect to the initial content of vitamin C was remarkably lowered. It was judged that this resulted from the fact that the compound having a $N_O/N_C$ value of greater than 0.2 was used as the oil phase in Comparative Example 1.

**Claims**

**1.** A cosmetic composition comprising:

an aqueous phase comprising a physiologically active antioxidant; and
an oil phase coming into contact with the entire upward surface layer of the aqueous phase, wherein the surface layer of the aqueous phase and a lower layer of the oil phase come into contact with each other to form an interface therebetween.

2. The cosmetic composition of claim 1, wherein the surface layer of the aqueous phase is covered with the lower layer of the oil phase to inhibit oxidation of the physiologically active antioxidant in the aqueous phase.

3. The cosmetic composition of claim 1, wherein the aqueous phase and the oil phase each independently comprise 10% by weight or less of a surfactant, based on the total weight of the aqueous phase or the oil phase.

4. The cosmetic composition of claim 1, wherein the oil phase has an oxygen solubility of 4.5 $\mu$mol/g or less at 1 atmospheric pressure (atm) and 25°C.

5. The cosmetic composition of claim 4, wherein an oil of the oil phase satisfies the following Expression 1:

$$[Expression\ 1]$$

$$N_O/N_C \leq 0.1$$

(wherein $N_O$ represents the number of oxygen atoms in the molecule, and $N_C$ represents the number of carbon atoms in the molecule).

6. The cosmetic composition of claim 1, wherein the physiologically active antioxidant comprises vitamin C or a derivative thereof.

7. The cosmetic composition of claim 6, wherein the vitamin C or the derivative thereof comprises any one or two more selected from ascorbic acid; a water-soluble derivative of ascorbic acid; a capsule having ascorbic acid collected therein; and a capsule having a water-soluble derivative of ascorbic acid collected therein.

8. The cosmetic composition of claim 1, wherein the physiologically active antioxidant comprises any one or two more selected from a carotenoid-based compound, a flavonoid-based compound, an isoflavone-based compound, and a water-soluble vitamin-based compound.

9. The cosmetic composition of claim 1, wherein the oil phase further comprises a fat-soluble derivative of vitamin C.

10. Double-layer cosmetics comprising the cosmetic composition of any one of claims 1 to 9 accommodated in a case,
wherein the aqueous phase comes into contact with an inner bottom of the case, and one side of the aqueous phase comes into contact with an inner side of the case, and
the entire upward surface layer of the aqueous phase is covered with the oil phase to inhibit oxidation of the physiologically active antioxidant in the aqueous phase.

11. The double-layer cosmetics of claim 10, wherein a material of the case is glass.

12. A method for inhibiting oxidation of a physiologically active antioxidant, the method comprising the steps of:

Disposing an oil phase on an aqueous phase comprising a physiologically active antioxidant, wherein the oxidation of the physiologically active antioxidant in the aqueous phase is inhibited by blocking the physiologically active antioxidant from coming into contact with oxygen in the atmosphere since a surface layer of the aqueous phase and a lower layer of the oil phase come into contact with each other to form an interface therebetween.

13. The method of claim 12, wherein the oil phase has an oxygen solubility of 4.5 $\mu$mol/g or less at 1 atm and 25°C.

14. The method of claim 13, wherein an oil of the oil phase satisfies the following Expression 1:

$$[Expression\ 1]$$

$$N_O/N_C \leq 0.1$$

(wherein $N_O$ represents the number of oxygen atoms in the molecule, and $N_C$ represents the number of carbon atoms in the molecule).

【FIG. 1】

## INTERNATIONAL SEARCH REPORT

| International application No. |
|---|
| **PCT/KR2018/009918** |

**A.  CLASSIFICATION OF SUBJECT MATTER**

*A61K 8/02(2006.01)i, A61K 8/92(2006.01)i, A61K 8/67(2006.01)i, A61Q 19/08(2006.01)i*

According to International Patent Classification (IPC) or to both national classification and IPC

**B.  FIELDS SEARCHED**

Minimum documentation searched (classification system followed by classification symbols)
A61K 8/02; A61K 7/00; A61K 7/42; A61K 8/03; A61K 8/06; A61K 8/11; A61K 8/34; A61K 8/37; A61K 8/67; A61Q 1/04; A61K 8/92; A61Q 19/08

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: bioactive, antioxidant, cosmetic composition, double layer, vitamin C, oil phase, aqueous phase, interface, double layer, ascorbic acid, air cut, protection, oxidization inhibition

**C.  DOCUMENTS CONSIDERED TO BE RELEVANT**

| Category* | Citation of document, with indication, where appropriate, of the relevant passages | Relevant to claim No. |
|---|---|---|
| X | KR 10-2013-0060663 A (COWAY CO., LTD.) 10 June 2013<br>See abstract; paragraphs [0026], [0034], [0035], [0045], [0047]-[0049], [0058], [0065]; claims 1, 2; and figure 1. | 1-14 |
| A | US 6019991 A (TANAKA, T. et al.) 01 February 2000<br>See the entire document. | 1-14 |
| A | JP 2016-124817 A (KAO CORP.) 11 July 2016<br>See the entire document. | 1-14 |
| A | KR 10-2015-0138681 A (COREANA COSMETICS CO., LTD.) 10 December 2015<br>See the entire document. | 1-14 |
| A | KR 10-2007-0018459 A (LG HOUSEHOLD & HEALTH CARE LTD.) 14 February 2007<br>See the entire document. | 1-14 |
| A | JP 2002-193742 A (NARIS COSMETICS CO., LTD.) 10 July 2002<br>See the entire document. | 1-14 |
| PX | KR 10-1833040 B1 (DAEDUCK LAB CO., LTD.) 27 February 2018<br>See claims 1-18. | 1-14 |

☐ Further documents are listed in the continuation of Box C.       ☒ See patent family annex.

| | | |
|---|---|---|
| * | Special categories of cited documents: | "T"  later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X"  document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&"  document member of the same patent family |

| Date of the actual completion of the international search | Date of mailing of the international search report |
|---|---|
| 05 DECEMBER 2018 (05.12.2018) | **05 DECEMBER 2018 (05.12.2018)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2018/009918**

| Patent document cited in search report | Publication date | Patent family member | Publication date |
|---|---|---|---|
| KR 10-2013-0060663 A | 10/06/2013 | NONE | |
| US 6019991 A | 01/02/2000 | NONE | |
| JP 2016-124817 A | 11/07/2016 | NONE | |
| KR 10-2015-0138681 A | 10/12/2015 | NONE | |
| KR 10-2007-0018459 A | 14/02/2007 | NONE | |
| JP 2002-193742 A | 10/07/2002 | JP 4450986 B2 | 14/04/2010 |
| KR 10-1833040 B1 | 27/02/2018 | NONE | |

Form PCT/ISA/210 (patent family annex) (January 2015)